Europäisches Patentamt

European Patent Office

Office européen des brevets

(19)

(11) **EP 1 159 246 B1**

(12) **EUROPÄISCHE PATENTSCHRIFT**

(45) Veröffentlichungstag und Bekanntmachung des Hinweises auf die Patenterteilung:
**15.10.2003 Patentblatt 2003/42**

(21) Anmeldenummer: **00910722.8**

(22) Anmeldetag: **28.02.2000**

(51) Int Cl.⁷: **C07C 51/25**, C07C 57/04

(86) Internationale Anmeldenummer:
**PCT/EP00/01633**

(87) Internationale Veröffentlichungsnummer:
**WO 00/053559 (14.09.2000 Gazette 2000/37)**

(54) **VERFAHREN DER KATALYTISCHEN GASPHASENOXIDATION VON ACROLEIN ZU ACRYLSÄURE**

METHOD FOR THE CATALYTIC GAS PHASE OXIDATION OF ACROLEIN INTO ACRYLIC ACID

PROCEDE D'OXYDATION CATALYTIQUE EN PHASE GAZEUSE D'ALDEHYDE ACRYLIQUE POUR FORMER DE L'ACIDE ACRYLIQUE

(84) Benannte Vertragsstaaten:
**BE DE ES FR IT**

(30) Priorität: **10.03.1999 DE 19910508**

(43) Veröffentlichungstag der Anmeldung:
**05.12.2001 Patentblatt 2001/49**

(73) Patentinhaber: **BASF AKTIENGESELLSCHAFT**
**67056 Ludwigshafen (DE)**

(72) Erfinder:
• **UNVERRICHT, Signe**
**D-68169 Mannheim (DE)**
• **ARNOLD, Heiko**
**D-67056 Ludwigshafen (DE)**
• **TENTEN, Andreas**
**D-67487 Maikammer (DE)**
• **HAMMON, Ulrich**
**D-68165 Mannheim (DE)**

(56) Entgegenhaltungen:
EP-A- 0 253 409      EP-A- 0 293 224
EP-A- 0 534 294      DE-A- 2 513 405
DE-A- 4 431 949

**Beschreibung**

Vorliegende Erfindung betrifft ein Verfahren der katalytischen Gasphasenoxidation von Acrolein zu Acrylsäure, bei dem man ein Acrolein, molekularen Sauerstoff und wenigstens ein Inertgas, das zu wenigstens 20 % seines Volumens aus molekularem Stickstoff besteht, enthaltendes Reaktionsgasausgangsgemisch, das den molekularen Sauerstoff und das Acrolein in einem molaren Verhältnis $O_2:C_3H_4O \geq 0,5$ enthält, so über einen Festbettkatalysator, dessen Aktivmasse wenigstens ein Molybdän und Vanadin enthaltendes Multimetalloxid ist, führt, daß der Acroleinumsatz bei einmaligem Durchgang $\geq 90$ mol-% und die damit einhergehende Selektivität der Acrylsäurebildung $\geq 90$ mol-% betragen.

Das vorgenannte Verfahren der katalytischen Gasphasenoxidation von Acrolein zu Acrylsäure ist allgemein bekannt (vgl. z.B. EP-A 714700 o. EP-A 700893, sowie die in diesen Schriften zitierte Literatur) und insbesondere als zweite Oxidationsstufe bei der Herstellung von Acrylsäure durch zweistufige katalytische Gasphasenoxidation ausgehend von Propen von Bedeutung. Acrylsäure ist ein bedeutendes Monomeres, das als solches oder in Form eines Alkylesters zur Erzeugung von z.B. als Klebstoffen geeigneten Polymerisaten Verwendung findet.

Die Zielsetzung einer jeden katalytischen Festbettgasphasenoxidation von Acrolein zu Acrylsäure besteht grundsätzlich darin, eine möglichst hohe Raum-Zeit-Ausbeute (RZA) an Wertprodukt zu erzielen (das ist bei einer kontinuierlichen Verfahrensweise die je Stunde und Volumen der verwendeten Katalysatorschüttung in Litern erzeugte Menge an Acrylsäure).

Es besteht deshalb generelles Interesse daran, die Gasphasenoxidation unter einer möglichst hohen Belastung der Katalysatorschüttung mit Acrolein (darunter wird die Menge an Acrolein in Normlitern (=Nl; das Volumen in Liter, das die entsprechende Acroleinmenge bei Normalbedingungen, d.h., bei 25°C und 1 bar, einnehmen würde) verstanden, die als Bestandteil des Reaktionsgasgemisches pro Stunde durch einen Liter an Katalysatorschüttung geführt wird) durchzuführen, ohne dabei den bei einmaligem Durchgang des Reaktionsausgangsgemisches durch die Katalysatorschüttung erfolgenden Umsatz an Acrolein sowie die Selektivität der damit einhergehenden Wertproduktbildung nennenswert zu beeinträchtigen.

Die Umsetzung des Vorgenannten wird durch die Tatsache beeinträchtigt, daß die Gasphasenoxidation von Acrolein zu Acrylsäure einerseits stark exotherm verläuft und andererseits von einer Vielfalt möglicher Parallel- und Folgereaktionen begleitet wird.

Mit zunehmender Acroleinbelastung der Katalysatorschüttung muß, bei Verwirklichung der angestrebten Randbedingung eines im wesentlichen gleichbleibenden Acroleinumsatzes, daher davon ausgegangen werden, daß, infolge der erhöhten lokalen Wärmeproduktion die Selektivität der Wertproduktbildung abnimmt.

Die konventionellen Verfahren der katalytischen Gasphasenoxidation von Acrolein zu Acrylsäure, die dadurch charakterisiert sind, daß als ein Hauptbestandteil des inerten Verdünnungsgases Stickstoff und außerdem ein in einer Reaktionszone befindlicher und längs dieser Reaktionszone homogener, d.h., über die Katalysatorschüttung chemisch einheitlich zusammengesetzter, Festbettkatalysator verwendet und die Temperatur der Reaktionszone auf einem über die Reaktionszone einheitlichen Wert gehalten wird (unter Temperatur einer Reaktionszone wird hier die Temperatur der in der Reaktionszone befindlichen Katalysatorschüttung bei Ausübung des Verfahrens in Abwesenheit einer chemischen Reaktion verstanden; ist diese Temperatur innerhalb der Reaktionszone nicht konstant, so meint der Begriff Temperatur einer Reaktionszone hier den Zahlenmittelwert der Temperatur der Katalysatorschüttung längs der Reaktionszone), beschränken daher den anzuwendenden Wert der Acroleinbelastung der Katalysatorschüttung auf Werte $\leq 150$ Nl Acrolein/l Katalysatorschüttung·h (vgl. z.B. EP-B 714700; dort beträgt die maximale angewandte Acroleinlast = 120 Nl Acrolein/l·h).

Die EP-B 253409 und das zugehörige Äquivalent, die EP-B 257565, offenbaren, daß bei Verwendung eines inerten Verdünnungsgases das eine höhere molare Wärmekapazität als molekularer Stickstoff aufweist, der Anteil an Propen im Reaktionsgasausgangsgemisch einer zweistufigen gasphasenkatalytischen Oxidation von Propen zu Acrylsäure erhöht werden kann. Nichtsdestotrotz liegt aber auch in den beiden vorgenannten Schriften die maximale realisierte Propenbelastung, und damit im wesentlichen automatisch auch eine' bei direktem Durchgang des Produktgasgemisches der Propenoxidationsstufe in die Acroleinoxidationsstufe nachfolgende Acroleinbelastung, der Katalysatorschüttung bei $\leq 140$ Nl Reaktand (Propen oder Acrolein)/l·h.

Lediglich in der EP-A 293224 wurden bisher Acroleinbelastungen oberhalb von 150 Nl Acrolein/l·h realisiert. Dies allerdings auf Kosten eines speziellen zu verwendenden inerten Verdünnungsgases, das völlig frei von molekularem Stickstoff ist. Nachteilig an diesem Verdünnungsgas ist insbesondere, daß es sich bei all seinen Bestandteilen, im Unterschied zu molekularem Stickstoff, um Wertprodukte handelt, die bei einer kontinuierlichen Durchführung des Verfahrens in aufwendiger Weise aus Gründen der Wirtschaftlichkeit wenigstens teilweise in die Gasphasenoxidation rückgeführt werden müssen.

Die Aufgabe der vorliegenden Erfindung bestand daher darin, ein wie Eingangs definiertes Verfahren der katalytischen Gasphasenoxidation von Acrolein zu Acrylsäure zur Verfügung zu stellen, das eine erhöhte Raum-Zeit-Ausbeute an Acrylsäure gewährleistet, ohne die Nachteile der Hochlastfahrweisen des Standes der Technik aufzuweisen.

Demgemäß wurde ein Verfahren der katalytischen Gasphasenoxidation von Acrolein zu Acrylsäure, bei dem man ein Acrolein, molekularen Sauerstoff und wenigstens ein Inertgas, das zu wenigstens 20 % seines Volumens aus molekularem Stickstoff besteht, enthaltendes Reaktionsgasausgangsgemisch, das den molekularen Sauerstoff und das Acrolein in einem molaren Verhältnis $O_2:C_3H_4O \geq 0,5$ enthält, bei erhöhter Temperatur so über einen Festbettkatalysator, dessen Aktivmasse wenigstens ein Molybdän und Vanadin enthaltendes Multimetalloxid ist, führt, daß der Acroleinumsatz bei einmaligem Durchgang ≥ 90 mol-% und die damit einhergehende Selektivität der Acrylsäurebildung ≥ 90 mol-% betragen, gefunden, das dadurch gekennzeichnet ist, daß

a) die Belastung des Festbettkatalysators mit dem im Reaktionsgasausgangsgemisch enthaltenen Acrolein > 150 Nl Acrolein/l Katalysatorschüttung · h beträgt,

b) der Festbettkatalysator aus einer in zwei räumlich aufeinanderfolgenden Reaktionszonen A,B angeordneten Katalysatorschüttung besteht, wobei die Temperatur der Reaktionszone A 230 bis 270°C und die Temperatur der Reaktionszone B 250 bis 300°C beträgt und gleichzeitig wenigstens 5°C oberhalb der Temperatur der Reaktionszone A liegt,

c) das Reaktionsgasausgangsgemisch die Reaktionszonen A,B in der zeitlichen Abfolge "erst A", "dann B" durchströmt und

d) sich die Reaktionszone A bis zu einem Umsatz des Acroleins von 55 bis 85 mol-% erstreckt.

Bevorzugt erstreckt sich die Reaktionszone A bis zu einem Acroleinumsatz von 65 bis 80 mol-%. Außerdem liegt die Temperatur der Reaktionszone A mit Vorteil bei 245 bis 260°C. Die Temperatur der Reaktionszone B liegt vorzugsweise wenigstens 10°C, besonders vorteilhaft 20°C oberhalb der Temperatur der Reaktionszone A und beträgt vorteilhaft 265 bis 285°C.

Je höher die Acroleinbelastung der Katalysatorschüttung beim erfindungsgemäßen Verfahren gewählt wird, um so größer sollte die Differenz zwischen der Temperatur der Reaktionszone A und der Temperatur der Reaktionszone B gewählt werden. Normalerweise wird die vorgenannte Temperaturdifferenz beim erfindungsgemäßen Verfahren aber nicht mehr als 40°C betragen. D.h., die Differenz zwischen der Temperatur der Reaktionszone A und der Temperatur der Reaktionszone B kann erfindungsgemäß bis zu 15°C, bis zu 25°C, bis zu 30°C, bis zu 35°C oder bis zu 40°C betragen.

Im übrigen kann der auf den einfachen Durchgang bezogene Acroleinumsatz beim erfindungsgemäßen Verfahren ≥ 92 mol-%, oder ≥ 94 mol-%, oder ≥ 96 mol-%, oder ≥ 98 mol-% und häufig sogar ≥ 99 mol-% betragen. Die Selektivität der Wertproduktbildung wird dabei regelmäßig ≥ 92 mol-%, bzw. ≥ 94 mol-%, häufig ≥ 95 mol-% oder ≥ 96 mol-% bzw. ≥ 97 mol-% betragen.

In überraschender Weise gilt das Vorgenannte nicht nur bei Acroleinbelastungen der Katalysatorschüttung von ≥ 150 Nl/l·h oder von ≥ 160 Nl/l·h bzw. ≥ 170 Nl/l·h oder ≥ 175 Nl/l·h bzw. ≥ 180 Nl/l·h, sondern auch bei Acroleinbelastungen der Katalysatorschüttung von ≥ 185 Nl/l·h oder von ≥ 190 Nl/l·h bzw. ≥ 200 Nl/l·h oder ≥ 210 Nl/l·h sowie bei Belastungswerten ≥ 220 Nl/l·h oder ≥ 230 Nl/l·h bzw. ≥ 240 Nl/l·h oder ≥ 250 Nl/l·h.

Dabei überrascht, daß vorgenannte Werte selbst dann erreichbar sind, wenn das erfindungsgemäß verwendete Inertgas zu ≥ 30 Vol.-%, oder zu ≥ 40 Vol.-%, oder zu ≥ 50 Vol.-%, oder zu ≥ 60 Vol.-%, oder zu ≥ 70 Vol.-%, oder zu ≥ 80 Vol.-%, oder zu ≥ 90 Vol.-%, oder zu ≥ 95 Vol.-% aus molekularem Stickstoff besteht.

In zweckmäßiger Weise wird das inerte Verdünnungsgas beim erfindungsgemäßen Verfahren zu 5 bis 20 Gew.-% aus $H_2O$ und zu 70 bis 90 Vol.-% aus $N_2$ bestehen.

Außer den in dieser Schrift genannten Bestandteilen enthält das Reaktionsgasausgangsgemisch normalerweise im wesentlichen keine weiteren Komponenten.

Bei Acroleinbelastungen oberhalb von 250 Nl/l·h wird für das erfindungsgemäße Verfahren die Mitverwendung von inerten (inerte Verdünnungsgase sollen generell solche sein, die sich beim einmaligem Durchgang zu weniger als 5 %, bevorzugt zu weniger als 2 % umsetzen) Verdünnungsgasen wie Propan, Ethan, Methan, Butan, Pentan, $CO_2$, CO, Wasserdampf und/oder Edelgasen empfohlen. Selbstverständlich können diese Gase aber auch bereits bei geringeren Belastungen mitverwendet werden. Auch ist die Anwendung eines nur aus einem oder mehreren der vorgenannten Gase bestehenden Inertgas möglich. Ferner überrascht, daß das erfindungsgemäße Verfahren mit einer über beide Reaktionszonen betrachtet homogenen, d.h., chemisch einheitlichen, Katalysatorschüttung durchgeführt werden kann, ohne in nennenswertem Umfang Umsatz- und/oder Selektivitätseinbußen zu erleiden.

Normalerweise wird beim erfindungsgemäßen Verfahren die Acroleinbelastung den Wert von 600 Nl/l·h nicht überschreiten. In typischer Weise liegen die Acroleinbelastungen beim erfindungsgemäßen Verfahren ohne nennenswerten Verlust von Umsatz und Selektivität bei Werten ≤ 300 Nl/l·h, häufig bei Werten ≤ 250 Nl/l·h.

Der Arbeitsdruck kann beim erfindungsgemäßen Verfahren sowohl unterhalb von Normaldruck (z.B. bis zu 0,5 bar) als auch oberhalb von Normaldruck liegen. Typischerweise wird der Arbeitsdruck bei Werten von 1 bis 5 bar, häufig 1 bis 3 bar liegen. Normalerweise wird der Reaktionsdruck 100 bar nicht überschreiten.

Das molare Verhältnis von $O_2$:Acrolein im Reaktionsgasausgangsgemisch muß erfindungsgemäß $\geq 0,5$ betragen. Häufig liegt er bei Werten $\geq 1$. Üblicherweise wird dieses Verhältnis bei Werten $\leq 3$ liegen. Häufig beträgt das molare Verhältnis von $O_2$:Acrolein im Reaktionsgasausgangsgemisch erfindungsgemäß 1 bis 2 bzw. 1 bis 1,5.

Als Quelle für den im Rahmen des erfindungsgemäßen Verfahrens erforderlichen molekularen Sauerstoff kommt sowohl Luft, als auch an molekularem Stickstoff entreicherte Luft (z.B. $\geq 90$ Vol.-% $O_2$, $\leq 10$ Vol.-% $N_2$) in Betracht.

Der Acroleinanteil im Reaktionsgasausgangsgemisch kann erfindungsgemäß z.B. bei Werten von 3 bis 15 Vol.-%, häufig bei 4 bis 10 Vol.-% bzw. 5 bis 8 Vol.-% liegen (jeweils bezogen auf das Gesamtvolumen).

Häufig wird man das erfindungsgemäße Verfahren bei einem Acrolein:Sauerstoff:Wasserdampf:Inertgas-Volumenverhältnis (Nl) von 1:(0,5 bzw. 1 bis 3):(0 bis 20):(3 bis 30), vorzugsweise von 1(1 bis 3):(0,5 bis 10):(7 bis 8) ausführen.

Normalerweise wird beim erfindungsgemäßen Verfahren Acrolein eingesetzt, das durch katalytische Gasphasenoxidation von Propen erzeugt wurde. In der Regel werden die Acrolein enthaltenden Reaktionsgase dieser Propenoxidation ohne Zwischenreinigung eingesetzt, weshalb das erfindungsgemäße Reaktionsgasausgangsgemisch auch geringe Mengen von z.B. nicht umgesetztem Propen oder von Nebenprodukten der Propenoxidation enthalten kann. Normalerweise muß dabei dem Produktgasgemisch der Propenoxidation noch der für die Acroleinoxidation erforderliche Sauerstoff zugesetzt werden.

Mit Vorteil wird eine solche dem erfindungsgemäßen Verfahren vorausgehende gasphasenkatalytische Oxidation von Propen zu Acrolein in Analogie zum erfindungsgemäßen Verfahren so durchgeführt, daß man ein Propen, molekularen Sauerstoff und wenigstens ein Inertgas, das zu wenigstens 20 % seines Volumens aus molekularem Stickstoff besteht, enthaltendes Reaktionsgasausgangsgemisch, das den molekularen Sauerstoff und das Propen in einem molaren Verhältnis $O_2$:$C_3H_6 \geq 1$ enthält, bei erhöhter Temperatur so über einen Festbettkatalysator, dessen Aktivmasse wenigstens ein Molybdän und/oder Wolfram sowie Wismut, Tellur, Antimon, Zinn und/oder Kupfer enthaltendes Multimetalloxid ist, führt, daß der Propenumsatz bei einmaligem Durchgang $\geq 90$ mol-% und die damit einhergehende Selektivität der Acroleinbildung sowie der Acrylsäurenebenproduktbildung zusammengenommen $\geq 90$ mol-% betragen, wobei,

a) die Belastung des Festbettkatalysators mit dem im Reaktionsgasausgangsgemisch enthaltenen Propen $\geq 160$ Nl Propen/l Katalysatorschüttung · h beträgt,

b) der Festbettkatalysator aus einer in zwei räumlich aufeinanderfolgenden Reaktionszonen A',B' angeordneten Katalysatorschüttung besteht, wobei die Temperatur der Reaktionszone A' 300 bis 330°C und die Temperatur der Reaktionszone B 300 bis 365°C beträgt und gleichzeitig wenigstens 5°C oberhalb der Temperatur der Reaktionszone A' liegt,

c) das Reaktionsgasausgangsgemisch die Reaktionszonen A',B' in der zeitlichen Abfolge "erst A'", "dann B'" durchströmt und

d) sich die Reaktionszone A' bis zu einem Umsatz des Propens von 40 bis 80 mol-% erstreckt.

Als Katalysatoren für die vorgenannte gasphasenkatalytische Propenoxidation kommen insbesondere jene der EP-A 15565, der EP-A 575897, der DE-A 19746210 und der DE-A 19855913 in Betracht.

Als Festbettkatalysatoren für die erfindungsgemäße gasphasenkatalytische Acroleinoxidation kommen alle diejenigen in Betracht, deren Aktivmasse wenigstens ein Mo und V enthaltendes Multimetalloxid ist. Solchermaßen geeignete Mulitmetalloxidkatalysatoren können beispielsweise der US-A 3 775 474, der US-A 3954855, der US-A 3893951 und der US-A 4339355 entnommen werden. Ferner eignen sich in besonderer Weise die Multimetalloxidmassen der EP-A 427508, der DE-A 2909671, der DE-C 3151805, der DE-AS 2626887, der DE-A 4302991, der EP-A 700893, der EP-A 714700 und der DE-A 19736105.

Besonders bevorzugt sind in diesem Zusammenhang die beispielhaften Ausführungsformen der EP-A 714700 sowie der DE-A 19736105.

Eine Vielzahl der erfindungsgemäß geeigneten Multimetalloxidaktivmassen läßt sich unter der allgemeinen Formel I

$$Mo_{12}V_aX^1{}_bX^2{}_cX^3{}_dX^4{}_eX^5{}_fX^6{}_gO_n \qquad (I),$$

in der die Variablen folgende Bedeutung haben:

$X^1 =$     W, Nb, Ta, Cr und/oder Ce,
$X^2 =$     Cu, Ni, Co, Fe, Mn und/oder Zn,
$X^3 =$     Sb und/oder Bi,
$X^4 =$     eines oder mehrere Alkalimetalle,
$X^5 =$     eines oder mehrere Erdalkalimetalle,
$X^6 =$     Si, Al, Ti und/oder Zr,
$a =$     1 bis 6,
$b =$     0,2 bis 4,
$c =$     0,5 bis 18,
$d =$     0 bis 40,
$e =$     0 bis 2,
$f =$     0 bis 4,
$g =$     0 bis 40 und
$n =$     eine Zahl, die durch die Wertigkeit und Häufigkeit der von Sauerstoff verschiedenen Elemente in I bestimmt wird,

subsummieren.

Bevorzugte Ausführungsformen innerhalb der aktiven Multimetalloxide I sind jene, die von nachfolgenden Bedeutungen der Variablen der allgemeinen Formel I erfaßt werden:

$X^1 =$     W, Nb und/oder Cr,
$X^2 =$     Cu, Ni, Co und/oder Fe,
$X^3 =$     Sb,
$X^4 =$     Na und/oder K,
$X^5 =$     Ca, Sr und/oder Ba,
$X^6 =$     Si, Al und/oder Ti,
$a =$     2,5 bis 5,
$b =$     0,5 bis 2,
$c =$     0,5 bis 3,
$d =$     0 bis 2,
$e =$     0 bis 0,2,
$f =$     0 bis 1 und
$n =$     eine Zahl, die durch die Wertigkeit und Häufigkeit der von Sauerstoff verschiedenen Elemente in I bestimmt wird.

Ganz besonders bevorzugte Multimetalloxide I sind jedoch jene der allgemeinen Formel I'

$$Mo_{12}V_{a'}Y^1{}_{b'}Y^2{}_{c'}Y^5{}_{f'}Y^6{}_{g'}O_{n'} \qquad (I')$$

mit

$Y^1 =$     W und/oder Nb,
$Y^2 =$     Cu und/oder Ni,
$Y^5 =$     Ca und/oder Sr,
$Y^6 =$     Si und/oder Al,
$a' =$     2 bis 4,
$b' =$     1 bis 1,5,
$c' =$     1 bis 3,
$f' =$     0 bis 0,5,
$g' =$     0 bis 8 und
$n' =$     eine Zahl, die durch die Wertigkeit und Häufigkeit der von Sauerstoff verschiedenen Elementen in I' bestimmt wird.

Die erfindungsgemäß geeigneten Multimetalloxidaktivmassen (I) sind in sich bekannter, z.B. in der DE-A 4335973 oder in der EP-A 714700 offenbarter, Weise erhältlich.

Prinzipiell können erfindungsgemäß geeignete Multimetalloxidaktivmassen, insbesondere solche der allgemeinen Formel I, in einfacher Weise dadurch hergestellt werden, daß man von geeigneten Quellen ihrer elementaren Konstituenten ein möglichst inniges, vorzugsweise feinteiliges, ihrer Stöchiometrie entsprechend zusammengesetztes, Trockengemisch erzeugt und dieses bei Temperaturen von 350 bis 600°C calciniert. Die Calcination kann sowohl unter Inertgas als auch unter einer oxidativen Atmosphäre wie z.B. Luft (Gemisch aus Inertgas und Sauerstoff) sowie auch unter reduzierender Atmosphäre (z.B. Gemische aus Inertgas und reduzierenden Gasen wie $H_2$, $NH_3$, CO, Methan und/oder Acrolein oder die genannten reduzierend wirkenden Gase für sich) durchgeführt werden. Die Calcinationsdauer kann einige Minuten bis einige Stunden betragen und nimmt üblicherweise mit der Temperatur ab. Als Quellen für die elementaren Konstituenten der Multimetalloxidaktivmassen I kommen solche Verbindungen in Betracht, bei denen es sich bereits um Oxide handelt und/oder um solche Verbindungen, die durch Erhitzen, wenigstens in Anwesenheit von Sauerstoff, in Oxide überführbar sind.

Das innige Vermischen der Ausgangsverbindungen zur Herstellung von Multimetalloxidmassen I kann in trockener oder in nasser Form erfolgen. Erfolgt es in trockener Form, so werden die Ausgangsverbindungen zweckmäßigerweise als feinteilige Pulver eingesetzt und nach dem Mischen und gegebenenfalls Verdichten der Calcinierung unterworfen. Vorzugsweise erfolgt das innige Vermischen jedoch in nasser Form.

Üblicherweise werden dabei die Ausgangsverbindungen in Form einer wäßrigen Lösung und/oder Suspension miteinander vermischt. Besonders innige Trockengemische werden beim beschriebenen Mischverfahren dann erhalten, wenn ausschließlich von in gelöster Form vorliegenden Quellen der elementaren Konstituenten ausgegangen wird. Als Lösungsmittel wird bevorzugt Wasser eingesetzt. Anschließend wird die erhaltene wäßrige Masse getrocknet, wobei der Trocknungsprozeß vorzugsweise durch Sprühtrocknung der wäßrigen Mischung mit Austrittstemperaturen von 100 bis 150°C erfolgt.

Die erfindungsgemäß geeigneten Multimetalloxidmassen, insbesondere jene der allgemeinen Formel I, können für das erfindungsgemäße Verfahren sowohl in Pulverform als auch zu bestimmten Katalysatorgeometrien geformt eingesetzt werden, wobei die Formgebung vor oder nach der abschließenden Calcination erfolgen kann. Beispielsweise können aus der Pulverform der Aktivmasse oder ihrer uncalcinierten Vorläufermasse durch Verdichten zur gewünschten Katalysatorgeometrie (z.B. durch Tablettieren, Extrudieren oder Strangpressen) Vollkatalysatoren hergestellt werden, wobei gegebenenfalls Hilfsmittel wie z.B. Graphit oder Stearinsäure als Gleitmittel und/oder Formhilfsmittel und Verstärkungsmittel wie Mikrofasern aus Glas, Asbest, Siliciumcarbid oder Kaliumtitanat zugesetzt werden können. Geeignete Vollkatalysatorgeometrien sind z.B. Vollzylinder oder Hohlzylinder mit einem Außendurchmesser und einer Länge von 2 bis 10 mm. Im Fall der Hohlzylinder ist eine Wandstärke von 1 bis 3 mm zweckmäßig. Selbstverständlich kann der Vollkatalysator auch Kugelgeometrie aufweisen, wobei der Kugeldurchmesser 2 bis 10 mm betragen kann.

Selbstverständlich kann die Formgebung der pulverförmigen Aktivmasse oder ihrer pulverförmigen, noch nicht calcinierten, Vorläufermasse auch durch Aufbringen auf vorgeformte inerte Katalysatorträger erfolgen. Die Beschichtung der Trägerkörper zur Herstellung der Schalenkatalysatoren wird in der Regel in einem geeigneten drehbaren Behälter ausgeführt, wie es z.B. aus der DE-A 2909671, der EP-A 293859 oder aus der EP-A 714700 bekannt ist.

Zweckmäßigerweise wird zur Beschichtung der Trägerkörper die aufzubringende Pulvermasse befeuchtet und nach dem Aufbringen, z.B. mittels heißer Luft, wieder getrocknet. Die Schichtdicke der auf den Trägerkörper aufgebrachten Pulvermasse wird zweckmäßigerweise im Bereich 10 bis 1000 μm, bevorzugt im Bereich 50 bis 500 μm und besonders bevorzugt im Bereich 150 bis 250 μm liegend, gewählt.

Als Trägermaterialien können dabei übliche poröse oder unporöse Aluminiumoxide, Siliciumdioxid, Thoriumdioxid, Zirkondioxid, Siliciumcarbid oder Silikate wie Magnesium- oder Aluminiumsilikat verwendet werden. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Geeignet ist die Verwendung von im wesentlichen unporösen, oberflächenrauhen, kugelförmigen Trägern aus Steatit, deren Durchmesser 1 bis 8 mm, bevorzugt 4 bis 5 mm beträgt. Geeignet ist aber auch die Verwendung von Zylindern als Trägerkörper, deren Länge 2 bis 10 mm und deren Außendurchmesser 4 bis 10 mm beträgt. Im Fall von erfindungsgemäß geeigneten Ringen als Trägerkörper liegt die Wanddicke darüber hinaus üblicherweise bei 1 bis 4 mm. Erfindungsgemäß bevorzugt zu verwendende ringförmige Trägerkörper besitzen eine Länge von 3 bis 6 mm, einen Außendurchmesser von 4 bis 8 mm und eine Wanddicke von 1 bis 2 mm. Erfindungsgemäß geeignet sind vor allem auch Ringe der Geometrie 7 mm x 3 mm x 4 mm (Außendurchmesser x Länge x Innendurchmesser) als Trägerkörper. Die Feinheit der auf die Oberfläche des Trägerkörpers aufzubringenden katalytisch aktiven Oxidmassen wird selbstredend an die gewünschte Schalendicke angepaßt (vgl. EP-A 714 700).

Günstige erfindungsgemäß zu verwendende Multimetalloxidaktivmassen sind ferner Massen der allgemeinen

Formel II,

$$[D]_p[E]_q \tag{II},$$

in der die Variablen folgende Bedeutung haben:

D = $Mo_{12}V_{a"}Z^1_{b"}Z^2_{c"}Z^3_{d"}Z^4_{e"}Z^5_{f"}Z^6_{g"}O_{x"}$ ,
E = $Z^7_{12}CU_{h"}H_{i"}O_{y"}$ ,
$Z^1$ = W, Nb, Ta, Cr und/oder Ce,
$Z^2$ = Cu, Ni, Co, Fe, Mn und/oder Zn,
$Z^3$ = Sb und/oder Bi,
$Z^4$ = Li, Na, K, Rb, Cs und/oder H,
$Z^5$ = Mg, Ca, Sr und/oder Ba,
$Z^6$ = Si, Al, Ti und/oder Zr,
$Z^7$ = Mo, W, V, Nb und/oder Ta,

a" = 1 bis 8,
b" = 0,2 bis 5,
c" = 0 bis 23,
d" = 0 bis 50,
e" = 0 bis 2,
f" = 0 bis 5,
g" = 0 bis 50,
h" = 4 bis 30,
i" = 0 bis 20 und
x",y" = Zahlen, die durch die Wertigkeit und Häufigkeit der von Sauerstoff verschiedenen Elemente in II bestimmt werden und
p,q = von Null verschiedene Zahlen, deren Verhältnis p/q 160:1 bis 1:1 beträgt,

und die dadurch erhältlich sind, daß man eine Multimetalloxidmasse E

$$Z^7_{12}Cu_{h"}H_{i"}O_{y"} \tag{E},$$

in feinteiliger Form getrennt vorbildet (Ausgangsmasse 1) und anschließend die vorgebildete feste Ausgangsmasse 1 in eine wäßrige Lösung, eine wäßrige Suspension oder in ein feinteiliges Trockengemisch von Quellen der Elemente Mo, V, $Z^1$, $Z^2$, $Z^3$, $Z^4$, $Z^5$, $Z^6$, die die vorgenannten Elemente in der Stöchiometrie D

$$Mo_{12}V_{a"}Z^1_{b"}Z^2_{c"}Z^3_{d"}Z^4_{e"}Z^5_{f"}Z^6_{g"} \tag{D},$$

enthält (Ausgangsmasse 2), im gewünschten Mengenverhältnis p:q einarbeitet, die dabei gegebenenfalls resultierende wäßrige Mischung trocknet, und die so gegebene trockene Vorläufermasse vor oder nach ihrer Trocknung zur gewünschten Katalysatorgeometrie bei Temperaturen von 250 bis 600°C calciniert.

Bevorzugt sind die Multimetalloxidmassen II, bei denen die Einarbeitung der vorgebildeten festen Ausgangsmasse 1 in eine wäßrige Ausgangsmasse 2 bei einer Temperatur ≤ 70°C erfolgt. Eine detaillierte Beschreibung der Herstellung von Multimetalloxidmassen II-Katalysatoren enthalten z.B. die EP-A 668104, die DE-A 19736105 und die DE-A 19528646.

Hinsichtlich der Formgebung gilt bezüglich Multimetalloxidmassen II-Katalysatoren das bei den Multimetalloxidmassen I-Katalysatoren Gesagte.

In anwendungstechnisch zweckmäßiger Weise erfolgt die Durchführung des erfindungsgemäßen Verfahrens in einem Zweizonenrohrbündelreaktor. Eine bevorzugt Variante eines erfindungsgemäß einsetzbaren Zweizonenrohrbündelreaktors offenbart die DE-C 2830765. Aber auch die in der DE-C 2513405, der US-A 3147084, der DE-A 2201528 und der DE-A 2903582 offenbarten Zweizonenrohrbündelreaktoren sind für eine Durchführung des erfindungsgemäßen Verfahrens geeignet.

D.h., in einfacher Weise befindet sich der erfindungsgemäß zu verwendende Festbettkatalysator in den Metallrohren eines Rohrbündelreaktors und um die Metallrohre werden zwei voneinander im wesentlichen räumlich getrennte Temperiermedien, in der Regel Salzschmelzen, geführt. Der Rohrabschnitt, über den sich das jeweilige Salzbad erstreckt, repräsentiert erfindungsgemäß eine Rekationszone.

D.h. in einfacher Weise umströmt ein Salzbad A diejenigen Abschnitte der Rohre (die Reaktionszone A), in welchem sich die oxidative Umsetzung des Acroleins (beim einfachen Durchgang) bis zum Erreichen eines Umsatzwertes im Bereich von 55 bis 85 mol.% vollzieht und ein Salzbad B umströmt den Abschnitt der Rohre (die Rekationszone B), in welchem sich die oxidative Anschlußumsetzung des Acroleins (beim einfachen Durchgang) bis zum Erreichen eines Umsatzwertes von wenigstens 90 mol.% vollzieht (bei Bedarf können sich an die erfindungsgemäß anzuwendenden Reaktionszonen A, B weitere Reaktionszonen anschließen, die auf individuellen Temperaturen gehalten werden).

Anwendungstechnisch zweckmäßig umfaßt das erfindungsgemäße Verfahren keine weiteren Reaktionszonen. D.h., das Salzbad B umströmt zweckmäßig den Abschnitt der Rohre, in welchem sich die oxidative Anschlußumsetzung des Acroleins (beim einfachen Durchgang) bis zu einem Umsatzwert von ≥ 92 mol.-%, oder ≥ 94 mol.-%, oder ≥ 96 mol.-%, oder ≥ 98 mol.-%, und häufig sogar ≥ 99 mol.-% oder mehr vollzieht.

Üblicherweise liegt der Beginn der Reaktionszone B hinter dem Heißpunktmaximum der Reaktionszone A. Die Temperatur des Heißpunktmaximums der Reaktionszone B liegt normalerweise unterhalb der Heißpunktmaximaltemperatur der Reaktionszone A.

Die beiden Salzbäder A, B können erfindungsgemäß relativ zur Strömungsrichtung des durch die Reaktionsrohre strömenden Reaktiongasgemisches im Gleichstrom oder im Gegenstrom durch den die Reaktionsrohre umgebenden Raum geführt werden. Selbstverständlich kann erfindungsgemäß auch in der Reaktionszone A eine Gleichströmung und in der Reaktionszone B eine Gegenströmung (oder umgekehrt) angewandt werden.

Selbstverständlich kann man in allen vorgenannten Fallkonstellationen innerhalb der jeweiligen Reaktionszone der, relativ zu den Reaktionsrohren, erfolgenden Parallelströmung der Salzschmelze noch eine Querströmung überlagern, so daß die einzelne Reaktionszone einem wie in der EP-A 700714 oder in der EP-A 700893 beschriebenen Rohrbündelreaktor entspricht und insgesamt im Längsschnitt durch das Kontaktrohrbündel ein mäanderförmiger Strömungsverlauf des Wärmeaustauschmittels resultiert.

Zweckmäßigerweise wird das Reaktionsgasausgangsgemisch der Katalysatorbeschickung auf die Reaktionstemperatur vorerwärmt zugeführt.

Üblicherweise sind in den vorgenannten Rohrbündelreaktoren die Kantaktrohre aus ferritischem Stahl gefertigt und weisen in typischer Weise eine Wanddicke von 1 bis 3 mm auf. Ihr Innendurchmesser beträgt in der Regel 20 bis 30 mm, häufig 22 bis 26 mm. Anwendungstechnisch zweckmäßig beläuft sich die im Rohrbündelbehälter untergebrachte Anzahl an Kontaktrohren auf wenigstens 5000, vorzugsweise auf wenigstens 10000. Häufig beträgt die Anzahl der im Reaktionsbehälter untergebrachten Kontaktrohre 15000 bis 30000. Rohrbündelreaktoren mit einer oberhalb von 40000 liegenden Anzahl an Kontaktrohren bilden eher die Ausnahme. Innerhalb des Behälters sind die Kontaktrohre im Normalfall homogen verteilt angeordnet, wobei die Verteilung zweckmäßig so gewählt wird, daß der Abstand der zentrischen Innenachsen von zueinander nächstliegenden Kontaktrohren (die sogenannte Kontaktrohrteilung) 35 bis 45 mm beträgt (vgl. EP-B 468290).

Als Wärmeaustauschmittel eignen sich insbesondere fluide Temperiermedien. Besonders günstig ist die Verwendung von Schmelzen von Salzen wie Kaliumnitrat, Kaliumnitrit, Natriumnitrit und/oder Natriumnitrat, oder von niedrig schmelzenden Metallen wie Natrium, Quecksilber sowie Legierungen verschiedener Metalle.

In der Regel wird bei allen vorstehend erwähnten Konstellationen der Stromführung in den Zweizonenrohrbündelreaktoren die Fließgeschwindigkeit innerhalb der beiden erforderlichen Wärmeaustauschmittelkreisläufe so gewählt, daß die Temperatur des Wärmeaustauschmittels von der Eintrittstelle in die Reaktionszone bis zur Austrittsstelle aus der Reaktionszone um 0 bis 15°C ansteigt. D.h., das vorgenannte ΔT kann erfindungsgemäß 1 bis 10°C, oder 2 bis 8°C oder 3 bis 6°C betragen.

Die Eintrittstemperatur des Wärmeaustauschmittels in die Reaktionszone A beträgt erfindungsgemäß normalerweise 230 bis 270°C. Die Eintrittstemperatur des Wärmeaustauschmittels in die Reaktionszone B beträgt erfindungsgemäß normalerweise einerseits 250°C bis 300°C und liegt andererseits gleichzeitig wenigstens 5°C oberhalb der Eintrittstemperatur des in die Reaktionszone A eintretenden Wärmeaustauschmittels.

Bevorzugt liegt die Eintrittstemperatur des Wärmeaustauschmittels in die Reaktionszone B wenigstens 10°C bzw. wenigstens 20°C oberhalb der Eintrittstemperatur des in die Reaktionszone A eintretenden Wärmeaustauschmittels. Die Differenz zwischen den Eintrittstemperaturen in die Reaktionszone A bzw. B kann erfindungsgemäß somit bis zu 15°C, bis zu 25°C, bis zu 30°C, bis zu 35°C oder bis zu 40°C betragen. Normalerweise wird die vorgenannte Temperatur aber nicht mehr als 50°C betragen. Je höher die Acroleinbelastung der Katalysatorschüttung beim erfindungsgemäßen Verfahren gewählt wird, um so größer sollte die Differenz zwischen der Eintrittstemperatur des Wärmeaustauschmittels in die Reaktionszone A und der Eintrittstemperatur des Wärmeaustauschmittels in die Reaktionszone B sein. Bevorzugt liegt die Eintrittstemperatur in die Reaktionszone A bei 245 bis 260°C und die Eintrittstemperatur in die Reaktionszone B bei 265 bis 285°C.

Selbstverständlich können beim erfindungsgemäßen Verfahren die beiden Reaktionszonen A, B auch in räumlich voneinander getrennten Rohrbündelreaktoren realisiert sein. Bei Bedarf kann zwischen den beiden Reaktionszonen A, B auch ein Wärmetauscher angebracht werden. Selbstredend können die beiden Reaktionszonen A, B auch als Wirbelbett gestaltet werden.

Ferner können beim erfindungsgemäßen Verfahren auch Katalysatorschüttungen verwendet werden, deren volumenspezifische Aktivität in Strömungsrichtung des Reaktionsgasgemisches kontinuierlich, abrupt oder stufenförmig zunimmt (dies kann z.B. durch Verdünnung mit Inertmaterial oder Variation der Aktivität des Multimetalloxids bewirkt werden).

Ebenso können für die beschriebene Zweizonenfahrweise auch die in der EP-A 293224 und in der EP-B 257565 empfohlenen inerten Verdünnungsgase (z.B. nur Propan, oder nur Methan etc.) eingesetzt werden. Letzteres bei Bedarf auch kombiniert mit einer in Strömungsrichtung des Reaktionsgasgemisches abnehmenden volumenspezifischen Aktivität der Katalysatorschüttung.

Es sei an dieser Stelle auch noch einmal darauf hingewiesen, daß für eine Durchführung des erfindungsgemäßen Verfahrens insbesondere auch der in der DE-AS 2201528 beschriebene Zweizonenrohrbündelreaktortyp verwendet werden kann, der die Möglichkeit beinhaltet, vom heißeren Wärmeaustauschmittel der Reaktionszone B eine Teilmenge an die Reaktionszone A abzuführen, um gegebenenfalls ein Anwärmen eines kalten Reaktionsgasausgangsgemisches oder eines kalten Kreisgases zu bewirken.

Das erfindungsgemäße Verfahren eignet sich insbesondere für eine kontinuierliche Durchführung. Es überrascht, daß es bei einmaligem Durchgang eine erhöhte Raum-Zeit-Ausbeute der Wertproduktbildung ermöglicht, ohne gleichzeitig die Selektivität der Wertproduktbildung nennenswert zu beeinträchtigen. Vielmehr wird in der Regel tendenziell sogar eine erhöhte Selektivität der Wertproduktbildung beobachtet. Letzteres ist vermutlich darauf zurückzuführen, daß das erfindungsgemäße Verfahren aufgrund der im Bereich des erhöhten Acroleinumsatzes vorliegenden erhöhten Temperaturen eine geringere Readsorption der gebildeten Acrylsäure an den Festbettkatalysator bedingt.

Bemerkenswert ist ferner, daß die Katalysatorlebensdauer beim erfindungsgemäßen Verfahren trotz der extremen Katalysatorbeladung mit Reaktanden im vollen Umfang zu befriedigen vermag.

Bei dem erfindungsgemäßen Verfahren wird keine reine Acrylsäure sondern ein Gemisch erhalten, von dessen Nebenkomponenten die Acrylsäure in an sich bekannter Weise (z.B. rektifikativ und/oder kristallisativ) abgetrennt werden kann. Nich umgesetztes Acrolein, Propen sowie verwendetes und/oder im Verlauf der Reaktion gebildetes inertes Verdünnungsgas können in die Gasphasenoxidation rückgeführt werden. Bei einer zweistufigen, von Propen ausgehenden Gasphasenoxidation erfolgt die Rückführung zweckmäßigerweise in die erste Oxidationsstufe. Natürlich kann die erfindungsgemäße Zweizonenfahrweise bei Bedarf auch im Fall konventioneller Propenlasten angewendet werden.

Im übrigen sind in dieser Schrift Umsatz, Selektivität und Verweilzeit, falls nichts anderes erwähnt wird, wie folgt definiert:

$$\text{Umsatz } U_A \text{ an Acrolein (\%)} = \frac{\text{Molzahl umgesetztes Acrolein}}{\text{Mohlzahl eingesetztes Acrolein}} \times 100$$

$$\text{Selektivität } S_A \text{ der Acrylsäurebildung (\%)} = \frac{\text{Mohlzahl Acrolein umgesetzt zu Acrylsäure}}{\text{Molzahl umgesetztes Acrolein}} \times 100$$

$$\text{Verweilzeit (sec.)} = \frac{\text{mit Katalysator gefülltes Leervolumen des Reaktors (l)}}{\text{durchgesetzte Menge Reaktionsgasausgangsgemisch (l/h)}} \times 3600$$

Beispiele:

a) Katalysatorherstellung

1. Herstellung der katalytisch aktiven Oxidmasse $Mo_{12}V_3W_{1,2}Cu_{2,4}O_n$

190 g Kupfer(II)acetatmonohydrat wurden in 2700 g Wasser zu einer Lösung I gelöst. In 5500 g Wasser wurden bei 95°C nacheinander 860 g Ammoniumheptamolybdattetrahydrat, 143 g Ammoniummetavanadat und 126 g Ammoniumparawolframatheptahydrat zu einer Lösung II gelöst. Anschließend wurde die Lösung I auf einmal in die Lösung II eingerührt und anschließend soviel einer 25gew.-%igen wäßrigen $NH_3$-Lösung zugesetzt, bis wieder eine Lösung entstand. Diese wurde bei einer Austrittstemperatur von 110°C sprühgetrocknet. Das resultierende Sprühpulver wurde je kg Pulver mit 0,25 kg einer 30gew.-%igen wäßrigen Essigsäurelösung mit einem Kneter der Fa. Werner & Pfleiderer vom Typ ZS1·80 verknetet und anschließend bei einer Temperatur von 110°C während 10 h im Trockenschrank getrocknet.

700 g des so erhaltenen Katalysatorvorläufers wurden in einem Luft/Stickstoffgemisch [(200 1N$_2$/15 l Luft)/h] in einem Drehrohrofen (50 cm lang, 12 cm Innendurchmesser) calciniert. Im Rahmen der Calcinierung wurde die Knetmasse zunächst innerhalb von einer Stunde von Raumtemperatur (ca. 25°C) kontinuierlich auf 325°C erhitzt. Anschließend wurde während 4 h auf dieser Temperatur gehalten. Dann wurde innerhalb von 15 min auf 400°C erwärmt, bei dieser Temperatur während 1 h gehalten und dann auf Raumtemperatur abgekühlt.

Das calcinierte katalytisch aktive Material wurde zu einem feinteiligen Pulver gemahlen, von dem 50 % der Pulverpartikel ein Sieb der Maschenweite 1 bis 10 µm passierten und dessen Anteil an Partikel mit einer Längstausdehnung oberhalb von 50 µm weniger als 1 % betrug.

b) Schalenkatalysatorherstellung

28 kg ringförmiger Trägerkörper (7 mm Außendurchmesser, 3 mm Länge, 4 mm Innendurchmesser, Steatit, mit einer Oberflächenrauhigkeit Rz gemäß EP-B 714700 von 45 µm und mit einem auf das Volumen der Trägerkörper bezogenen Porengesamtvolumen ≤ 1 Vol.-%, Hersteller: Caramtec DE) wurden in einem Dragierkessel (Neigungswinkel 90°; Hicoater der Fa. Lödige, DE) von 200 1 Innenvolumen gefüllt. Anschließend wurde der Dragierkessel mit 16 U/min in Rotation versetzt. Über eine Düse wurden innerhalb von 25 min 2000 g einer aus 75 Gew.-% H$_2$O und 25 Gew.-% Glycerin bestehenden wäßrigen Lösung auf die Trägerkörper aufgesprüht. Gleichzeitig wurden im selben Zeitraum 7 kg des katalytisch aktiven Oxidpulvers aus a) über eine Schüttelrinne außerhalb des Sprühkegels der Zerstäuberdüse kontinuierlich zudosiert. Während der Beschichtung wurde das zugeführte Pulver vollständig auf die Oberfläche der Trägerkörper aufgenommen, eine Agglomeration der feinteiligen oxidischen Aktivmasse wurde nich beobachtet. Nach beendeter Zugabe von Pulver und wäßriger Lösung wurde bei einer Drehgeschwindigkeit von 2 Umdrehungen/min 20 min. 110°C heiße Luft in den Dragierkessel geblasen. Anschließend wurde noch 2 h bei 250°C in ruhender Schüttung (Hordenofen) unter Luft getrocknet. Es wurden ringförmige Schalenkatalysatoren erhalten, deren Anteil an oxidischer Aktivmasse, bezogen auf die Gesamtmasse, 20 Gew.-% betrug. Die Schalendicke lag, sowohl über die Oberfläche eines Trägerkörpers als auch über die Oberfläche verschiedener Trägerkörper betrachtet, bei 230 ± 25 µm.

b) Gasphasenkatalytische Oxidation von Acrolein zu Acrylsäure

1. Beschickung des Reaktionsrohres

Ein Reaktionsrohr (V2A Stahl; 30 mm Außendurchmesser; 2 mm Wandstärke; 26 mm Innendurchmesser, Länge: 439 cm, sowie ein in der Reaktionsrohrmitte zentriertes Thermo-Rohr (4 mm Außendurchmesser) zur Aufnahme eines Thermoelements mit dem die Temperatur im Reaktionsrohr ermittelt werden kann) wurde von unten nach oben auf einem Kontaktstuhl (44 cm Länge) zunächst auf einer Länge von 30 cm mit eine rauhe Oberfläche aufweisenden Steatitkugeln (4 bis 5 mm Durchmesser; Inertmaterial zum Erwärmen des Reaktionsgasausgangsgemisches) und anschließend auf einer Länge von 300 cm mit den in a) hergestellten Schalenkatalysatorringen beschickt, bevor die Beschickung auf einer Länge von 30 cm mit den vorgenannten Steatitkugeln als Nachschüttung abgeschlossen wurde. Die verbleibenden 35 cm Kontaktrohr wurden leer belassen.

Der Teil des Reaktionsrohres, der mit Feststoff beschickt war, wurde mittels 12 zylinderförmig um das Rohr aufgegossenen Aluminium-Blöcken von je 30 cm Länge thermostatisiert (Vergleichsversuche mit einem entsprechenden mittels eines stickstoffgeperlten Salzbades beheizten Reaktionsrohr zeigten, daß die Aluminiumblock-Thermostatisierung eine Salzbad-Thermostatisierung zu simulieren vermag). Die ersten sechs Aluminiumblöcke in Strömungsrichtung definierten eine Reaktionszone A und die verbleibenden sechs Aluminiumblöcke definierten eine Reaktionszone B. Die an Feststoff freien Enden des Reaktionsrohres wurden mit unter Druck befindlichem Wasserdampf auf 220°C gehalten.

Das vorstehend beschriebene Reaktionsrohr wurde mit einem Reaktionsgasausgangsgemisch der nachfolgenden Zusammensetzung kontinuierlich beschickt, wobei die Belastung und die Thermostatisierung des Reaktionsrohres variiert wurden:

5,5 Vol.-% Acrolein,
0,3 Vol.-% Propen,
6,0 Vol.-% molekularer Sauerstoff,
0,4 Vol.-% CO,

0,8 Vol.-% $CO_2$,
9.0 Vol.-% Wasser und
78,0 Vol.-% molekularer Stickstoff.

Dem Produktgasgemisch wurde am Reaktionsrohrausgang eine kleine Probe für die gaschromatographische Analyse entnommen. Am Ende der Reaktionszone A befand sich ebenfalls eine Analysenstelle.

Die in Abhängigkeit von der gewählten Acroleinbelastung und der gewählten Aluminium-Thermostatisierung erzielten Ergebnisse zeigt die nachfolgende Tabelle 1.

$T_A$ steht für die Temperatur der Aluminiumblöcke in der Reaktionszone A und $T_B$ steht für die Temperatur der Aluminiumblöcke in der Reaktionszone B.

$U_{AA}$ ist der Acroleinumsatz am Ende der Reaktionszone A und $U_{AE}$ ist der Acroleinumsatz am Reaktionsrohrausgang. $S_{AE}$ ist die Selektivität der Acrylsäurebildung am Reaktionsrohrausgang und $RZA_A$ ist die Raum-Zeit-Ausbeute an Acrylsäure am Reaktionsrohrausgang.

Abschließend sei festgehalten, daß anstelle der im Beispiel verwendeten Katalysatorschüttung auch eine entsprechende Schüttung gemäß Beispiel 3 der DE-A 19736105 verwendet werden kann.

Tabelle 1

| Acroleinbelastung [Nl Acrolein/l·h] | $T_A$ [°C] | $T_B$ [°C] | $U_{AA}$ (%) | $U_{AE}$ (%) | $S_{AE}$ (%) | $RZA_A$ (g/l·h) |
|---|---|---|---|---|---|---|
| 87 | 255 | 255 | 91,5 | 99,2 | 95,7 | 265 |
| 113 | 262 | 262 | 91,7 | 99,3 | 95,3 | 345 |
| 150 | 267 | 267 | 93,2 | 99,3 | 95,0 | 452 |
| 150 | 254 | 271 | 76,1 | 99,3 | 95,8 | 457 |
| 171 | 255 | 276 | 73,2 | 99,3 | 95,7 | 523 |

Erhöht man die Acroleinlast auf Werte > 175 Nl Acrolein/l·h, so erhält man die Ergebnisse gemäß Tabelle 2.

Tabelle 2

| Acroleinbelastung [Nl Acrolein/l·h] | $T_A$ [°C] | $T_B$ [°C] | $U_{AA}$ (%) | $U_{AE}$ (%) | $S_{AE}$ (%) | $RZA_A$ (g/l·h) |
|---|---|---|---|---|---|---|
| 190 | 257 | 281 | 78,2 | 99,3 | 95,7 | 579 |
| 210 | 257 | 286 | 71,7 | 99,3 | 95,6 | 640 |

EP 1 159 246 B1

## EP 1 159 246 B1

**Patentansprüche**

1. Verfahren der katalytischen Gasphasenoxidation von Acrolein zu Acrylsäure, bei dem man ein Acrolein, molekularen Sauerstoff und wenigstens ein Inertgas, das zu wenigstens 20% seines Volumens aus molekularem Stickstoff besteht, enthaltendes Reaktionsgasausgangsgemisch, das den molekularen Sauerstoff und das Acrolein in einem molaren Verhältnis $O_2:C_3H_4O \geq 0,5$ enthält, bei erhöhter Temperatur so über einen Festbettkatalysator, dessen Aktivmasse wenigstens ein Molybdän und Vanadin enthaltendes Multimetalloxid ist, führt, daß der Acroleinumsatz bei einmaligem Durchgang $\geq 90$ mol-% und die damit einhergehende Selektivität der Acrylsäurebildung $\geq 90$ mol-% betragen, das **dadurch gekennzeichnet ist, daß**

   a) die Belastung des Festbettkatalysators mit dem im Reaktionsgasausgangsgemisch enthaltenen Acrolein $\geq 150$ Nl Acrolein/l Katalysatorschüttung h beträgt,

   b) der Festbettkatalysator aus einer in zwei räumlich aufeinanderfolgenden Reaktionszonen A,B angeordneten Katalysatorschüttung besteht, wobei die Temperatur der Reaktionszone A 230 bis 270°C und die Temperatur der Reaktionszone B 250 bis 300°C beträgt und gleichzeitig wenigstens 5°C oberhalb der Temperatur der Reaktionszone A liegt,

   c) das Reaktionsgasausgangsgemisch die Reaktionszonen A,B in der zeitlichen Abfolge "erst A", "dann B" durchströmt und

   d) sich die Reaktionszone A bis zu einem Umsatz des Acroleins von 55 bis 85 mol-% erstreckt.

2. Verfahren nach Anspruch 1, **dadurch gekennzeichnet, daß** sich die Reaktionszone A bis zu einem Umsatz des Acroleins von 65 bis 80 mol-% erstreckt.

3. Verfahren nach Anspruch 1 oder 2, **dadurch gekennzeichnet, daß** die Temperatur der Reaktionszone B wenigstens 20°C oberhalb der Reaktionszone A liegt.

4. Verfahren nach einem der Ansprüche 1 bis 3, **dadurch gekennzeichnet, daß** die Temperatur der Reaktionszone A 245 bis 260°C beträgt.

5. Verfahren nach einem der Ansprüche 1 bis 4, **dadurch gekennzeichnet, daß** die Temperatur der Reaktionszone B 265 bis 285°C beträgt.

6. Verfahren nach einem der Ansprüche 1 bis 5, **dadurch gekennzeichnet, daß** der Acroleinumsatz bei einmaligem Durchgang $\geq 94$ mol-% beträgt.

7. Verfahren nach einem der Ansprüche 1 bis 6, **dadurch gekennzeichnet, daß** die Selektivität der Acrylsäurebildung $\geq 94$ mol-% beträgt.

8. Verfahren nach einem der Ansprüche 1 bis 7, **dadurch gekennzeichnet, daß** die Acroleinbelastung der Kataly-satorschüttung $\geq 160$ Nl/l·h beträgt.

9. Verfahren nach einem der Ansprüche 1 bis 7, **dadurch gekennzeichnet, daß** die Acroleinbelastung der Kataly-satorschüttung $\geq 170$ Nl/l.h beträgt.

10. Verfahren nach einem der Ansprüche 1 bis 9, **dadurch gekennzeichnet, daß** das wenigstens eine Inertgas zu > 40 Vol.-% aus molekularem Stickstoff besteht.

11. Verfahren nach einem der Ansprüche 1 bis 10, **dadurch gekennzeichnet, daß** das wenigstens eine Inertgas Wasserdampf umfaßt.

12. Verfahren nach einem der Ansprüche 1 bis 11, **dadurch gekennzeichnet, daß** das wenigstens eine Inertgas $CO_2$ und/oder CO umfaßt.

13. Verfahren nach einem der Ansprüche 1 bis 12, **dadurch gekennzeichnet, daß** es bei einem Arbeitsdruck von 0,5 bis 3 bar durchgeführt wird.

**14.** Verfahren nach einem der Ansprüche 1 bis 13, **dadurch gekennzeichnet, daß** das molare Verhältnis $O_2$:Acrolein im Reaktionsgasausgangsgemisch 1 bis 2 beträgt.

**15.** Verfahren nach einem der Ansprüche 1 bis 14, **dadurch gekennzeichnet, daß** als Sauerstoffquelle Luft mitverwendet wird.

**16.** Verfahren nach einem der Ansprüche 1 bis 15, **dadurch gekennzeichnet, daß** der Acroleingehalt des Reaktionsgasausgangsgemisches 3 bis 15 Vol.-% beträgt.

**17.** Verfahren nach einem der Ansprüche 1 bis 15, **dadurch gekennzeichnet, daß** der Acroleingehalt des Reaktionsgasausgangsgemisches 5 bis 8 Vol.-% beträgt.

**18.** Verfahren nach einem der Ansprüche 1 bis 17, **dadurch gekennzeichnet, daß** die Aktivmasse des Festbettkatalysators wenigstens ein Multimetalloxid der allgemeinen Formel I

$$Mo_{12}V_aX^1{}_bX^2{}_cX^3{}_dX^4{}_eX^5{}_fX^6{}_gO_n \tag{I}$$

mit

$X^1 =$ W, Nb, Ta, Cr und/oder Ce,
$X^2 =$ Cu, Ni, Co, Fe, Mn und/oder Zn,
$X^3 =$ Sb und/oder Bi,
$X^4 =$ eines oder mehrere Alkalimetalle,
$X^5 =$ eines oder mehrere Erdalkalimetalle,
$X^6 =$ Si, Al, Ti und/oder Zr,
$a =$ 1 bis 6,
$b =$ 0,2 bis 4,
$c =$ 0,5 bis 18,
$d =$ 0 bis 40,
$e =$ 0 bis 2,
$f =$ 0 bis 4,
$g =$ 0 bis 40 und
$n =$ eine Zahl, die durch die Wertigkeit und Häufigkeit der von Sauerstoff verschiedenen Elemente in I bestimmt wird, ist.

**19.** Verfahren nach einem der Ansprüche 1 bis 17, **dadurch gekennzeichnet, daß** die Aktivmasse des Festbettkatalysators wenigstens ein Multimetalloxid der allgemeinen Formel II

$$[D]_p[E]_q \tag{II},$$

in der die Variablen folgende Bedeutung haben:

$D =$ $Mo_{12}V_{a''}Z^1{}_{b''}Z^2{}_{c''}Z^3{}_{d''}Z^4{}_{e''}Z^5{}_{f''}Z^6{}_{g''}O_{x''}$,
$E =$ $Z^7{}_{12}Cu_h{}_{''}H_{i''}O_{y''}$,
$Z^1 =$ W, Nb, Ta, Cr und/oder Ce,
$Z^2 =$ Cu, Ni, Co, Fe, Mn und/oder Zn,
$Z^3 =$ Sb und/oder Bi,
$Z^4 =$ Li, Na, K, Rb, Cs und/oder H,
$Z^5 =$ Mg, Co, Sr und/oder Ba,
$Z^6 =$ Si, Al, Ti und/oder Zr,
$Z^7 =$ Mo, W, V, Nb und/oder Ta,

$a'' =$ 1 bis 8,
$b'' =$ 0,2 bis 5,
$c'' =$ 0 bis 23,

d"  = 0 bis 50,

e"  = 0 bis 2,

f"  = 0 bis 5,

g"  = 0 bis 50,

h"  = 4 bis 30,

i"  = 0 bis 20 und

x",y" = Zahlen, die durch die Wertigkeit und Häufigkeit der von Sauerstoff verschiedenen Element in II bestimmt werden und

p,q  = von Null verschiedene Zahlen, deren Verhältnis p/q 160:1 bis 1:1 beträgt,

und dadurch erhältlich ist, daß man eine Multimetalloxidmasse (E)

$$Z^7{}_{12}Cu_{h"}H_{i"}O_{y"} \tag{E},$$

in feinteiliger Form getrennt vorbildet (Ausgangsmasse 1) und anschließend die vorgebildete feste Ausgangsmasse 1 in eine wäßrige Lösung, eine wäßrige Suspension oder in ein feinteiliges Trockengemisch von Quellen der Elemente Mo, V, $Z^1$, Z2, $Z^3$, $Z^4$, $Z^5$, $Z^6$, die die vorgenannten Elemente in der Stöchiometrie D

$$Mo_{12}V_{a"}Z^1{}_{b"}Z^2{}_{c"}Z^3{}_{d"}Z^4{}_{e"}Z^5{}_{f"}Z^6{}_{g"} \tag{D},$$

enthält (Ausgangsmasse 2), im gewünschten Mengenverhältnis p:q einarbeitet, die dabei gegebenenfalls resultierende wäßrige Mischung trocknet, und die so erhaltene trockene Vorläufermasse vor oder nach ihrer Trocknung zur gewünschten Katalysatorgeometrie bei Temperaturen von 250 bis 600°C calciniert.

**20.** Verfahren nach einem der Ansprüche 1 bis 19, **dadurch gekennzeichnet, daß** die Katalysatorschüttung ringförmige Katalysatoren umfaßt.

**21.** Verfahren nach einem der Ansprüche 1 bis 19, **dadurch gekennzeichnet, daß** die Katalysatorschüttung kugelförmige Katalysatoren umfaßt.

**22.** Verfahren nach einem der Ansprüche 1 bis 21, **dadurch gekennzeichnet, daß** es in einem Zweizonenrohrbündelreaktor durchgeführt wird.

**23.** Verfahren nach einem der Ansprüche 1 bis 22, **dadurch gekennzeichnet, dass** die Temperatur der Reaktionszone B wenigstens 10°C oberhalb der Temperatur der Reaktionszone A liegt.

**24.** Verfahren nach einem der Ansprüche 1 bis 23, **dadurch gekennzeichnet, dass** das Reaktionsgasausgangsgemisch den molekularen Sauerstoff und das Acrolein in einem molaren Verhältnis $O_2$:$C_3H_4O \geq 1$ enthält.

**25.** Verfahren nach einem der Ansprüche 1 bis 24, **dadurch gekennzeichnet, dass** der Festbettkatalysator eine Katalysatorschüttung ist, deren volumenspezifische Aktivität in Strömungsrichtung durch Verdünnung mit Inertmaterial des Reaktionsgasgemisches kontinuierlich, abrupt oder stufenförmig zunimmt.

**Claims**

**1.** A process for the catalytic gas-phase oxidation of acrolein to acrylic acid, in which a reaction gas starting mixture comprising acrolein, molecular oxygen and at least one inert gas, at least 20% by volume of which consists of molecular nitrogen, and containing the molecular oxygen and the acrolein in a molar ratio $O_2$:$C_3H_4O \geq 0.5$ is passed, at elevated temperatures, over a fixed-bed catalyst, whose active material is at least one molybdenum- and vanadium-containing multimetal oxide, in such a way that the acrolein conversion in a single pass is $\geq$ 90 mol% and the associated selectivity of the acrylic acid formation is $\geq$ 90 mol%, wherein

 a) the loading of the fixed-bed catalyst where the acrolein contained in the reaction gas starting mixture is $\geq$ 150 1 (s.t.p.) of acrolein per 1 of catalyst bed per h,

b) the fixed-bed catalyst consists of a catalyst bed arranged in two spatially successive reaction zones A, B, the temperature of the reaction zone A being from 230 to 270°C and the temperature of the reaction zone B being from 250 to 300°C and at the same time being at least 5°C above the temperature of the reaction zone A,

c) the reaction gas starting mixture flows first through the reaction zone A and then through the reaction zone B and

d) the reaction zone A extends to an acrolein conversion of from 55 to 85 mol%.

2. A process as claimed in claim 1, wherein the reaction zone A extends to an acrolein conversion of from 65 to 80 mol%.

3. A process as claimed in claim 1 or 2, wherein the temperature of the reaction zone B is at least 20°C above that of the reaction zone A.

4. A process as claimed in any of claims 1 to 3, wherein the temperature of the reaction zone A is from 245 to 260°C.

5. A process as claimed in any of claims 1 to 4, wherein the temperature of the reaction zone B is from 265 to 285°C.

6. A process as claimed in any of claims 1 to 5, wherein the acrolein conversion in a single pass is $\geq$ 94 mol%.

7. A process as claimed in any of claims 1 to 6, wherein the selectivity of the acrylic acid formation is $\geq$ 94 mol%.

8. A process as claimed in any of claims 1 to 7, wherein the acrolein loading of the catalyst bed is $\geq$ 160 1 (s.t.p.)/l·h.

9. A process as claimed in any of claims 1 to 7, wherein the acrolein loading of the catalyst bed is $\geq$ 170 1 (s.t.p.)/l·h.

10. A process as claimed in any of claims 1 to 9, wherein the one or more inert gases comprise $\geq$ 40% by volume of molecular nitrogen.

11. A process as claimed in any of claims 1 to 10, wherein the one or more inert gases comprise steam.

12. A process as claimed in any of claims 1 to 11, wherein the one or more inert gases comprise $CO_2$ and/or CO.

13. A process as claimed in any of claims 1 to 12, which is carried out at an operating pressure of from 0.5 to 3 bar.

14. A process as claimed in any of claims 1 to 13, wherein the molar $O_2$:acrolein ratio in the reaction gas starting mixture is from 1 to 2.

15. A process as claimed in any of claims 1 to 14, wherein air is concomitantly used as an oxygen source.

16. A process as claimed in any of claims 1 to 15, wherein the acrolein content of the reaction gas starting mixture is from 3 to 15% by volume.

17. A process as claimed in any of claims 1 to 15, wherein the acrolein content of the reaction gas starting mixture is from 5 to 8% by volume.

18. A process as claimed in any of claims 1 to 17, wherein the active material of the fixed-bed catalyst is at least one multimetal oxide of the formula I

$$Mo_{12}V_aX^1_bX^2_cX^3_dX^4_eX^5_fX^6_gO_n \tag{I}$$

where

$X^1$    is W, Nb, Ta, Cr and/or Ce,
$X^2$    is Cu, Ni, Co, Fe, Mn and/or Zn,

$X^3$ is Sb and/or Bi,
$X^4$ is one or more alkali metals,
$X^5$ is one or more alkaline earth metals,
$X^6$ is Si, Al, Ti and/or Zr,
a is from 1 to 6,
b is from 0.2 to 4,
c is from 0.5 to 18,
d is from 0 to 40,
e is from 0 to 2,
f is from 0 to 4,
g is from 0 to 40 and
n is a number which is determined by the valency and frequency of the elements in I other than oxygen.

19. A process as claimed in any of claims 1 to 17, wherein the active material of the fixed-bed catalyst is at least one multimetal oxide of the formula II

$$[D]_p[E]_q \qquad (II),$$

where

D is $Mo_{12}V_{a"}Z^1_{b"}Z^2_{c"}Z^3_{d"}Z^4_{e"}Z^5_{f"}Z^6_{g"}O_{x"}$,
E is $Z^7_{12}Cu_{h"}H_{i"}O_{y"}$,
$Z^1$ is W, Nb, Ta, Cr and/or Ce,
$Z^2$ is Cu, Ni, Co, Fe, Mn and/or Zn,
$Z^3$ is Sb and/or Bi,
$Z^4$ is Li, Na, K, Rb, Cs and/or H
$z^5$ is Mg, Co, Sr and/or Ba,
$Z^6$ is Si, Al, Ti and/or Zr,
$Z^7$ is Mo, W, V, Nb and/or Ta,

a" is from 1 to 8,
b" is from 0.2 to 5,
c" is from 0 to 23,
d" is from 0 to 50,
e" is from 0 to 2,
f" is from 0 to 5,
g" is from 0 to 50,
h" is from 4 to 30,
i" is from 0 to 20 and
x", y" are numbers which are determined by the valency and frequency of the elements in II other than oxygen and
p, q are numbers other than zero, whose ratio p/q is from 160:1 to 1:1,

which is obtainable by separately preforming a multimetal oxide material (E)

$$Z^7_{12}Cu_{h"}H_{i"}O_{y"} \qquad (E),$$

in finely divided form (starting material 1) and then incorporating the preformed solid starting material 1 into an aqueous solution, an aqueous suspension or a finely divided dry blend of sources of the elements Mo, V, $Z^1$, $Z^2$, $Z^3$, $Z^4$, $Z^5$ and $Z^6$, which contains the abovementioned elements in the stoichiometry D

$$Mo_{12}V_{a"}Z^1_{b"}Z^2_{c"}Z^3_{d"}Z^4_{e"}Z^5_{f"}Z^6_{g"} \qquad (D),$$

(starting material 2), in the desired ratio p:q, drying any resulting aqueous mixture and calcining the resulting dry precursor material before or after its drying to the desired catalyst geometry at from 250 to 600°C.

20. A process as claimed in any of claims 1 to 19, wherein the catalyst bed comprises annular catalysts.

21. A process as claimed in any of claims 1 to 19, wherein the catalyst bed comprises spherical catalysts.

22. A process as claimed in any of claims 1 to 21, which is carried out in a two-zone tube-bundle reactor.

23. A process as claimed in any of claims 1 to 22, wherein the temperature of the reaction zone B is at least 10°C above the temperature of the reaction zone A.

24. A process as claimed in any of claims 1 to 23, wherein the reaction gas starting mixture contains the molecular oxygen and the acrolein in a molar ratio $O_2:C_3H_4O \geq 1$.

25. A process as claimed in any of claims 1 to 24, wherein the fixed-bed catalyst is a catalyst bed whose volume-specific activity increases continuously, abruptly or stepwise in the direction of flow of the reaction gas mixture through dilution with inert material.

**Revendications**

1. Procédé d'oxydation catalytique en phase gazeuse d'acroléine pour former de l'acide acrylique, dans lequel on guide un mélange de départ de gaz réactionnels contenant de l'acroléine, de l'oxygène moléculaire et au moins un gaz inerte, qui est constitué pour au moins 20% de son volume d'azote moléculaire, mélange qui contient l'oxygène moléculaire et l'acroléine dans un rapport molaire de $O_2/C_3H_4O \geq 0,5$, à une température élevée par un catalyseur à lit fixe, dont la masse active est au moins un oxyde multimétallique contenant du molybdène et du vanadium, de façon que la conversion d'acroléine soit, pour un passage unique, $\geq 90$ moles % et la sélectivité ainsi développée de la formation d'acide acrylique soit $\geq 90$ moles %, **caractérisé en ce que**

   a) la charge du catalyseur à lit fixe par l'acroléine contenue dans le mélange de départ de gaz réactionnels est $\geq 150$ Nl d'acroléine/l de masse de catalyseur·h,
   b) le catalyseur à lit fixe est constitué d'une masse de catalyseur agencée dans deux zones réactionnelles A, B spatialement successives, la température de la zone réactionnelle A étant de 230 à 250°C et la température de la zone réactionnelle B de 250 à 300°C et simultanément d'au moins 5°C supérieure à la température de la zone réactionnelle A,
   c) le mélange de départ de gaz réactionnels traverse les zones réactionnelles A, B dans la succession dans le temps de "tout d'abord A", "ensuite B", et
   d) la zone réactionnelle A s'étend jusqu'à une conversion de l'acroléine de 55 à 85 moles %.

2. Procédé suivant la revendication 1, **caractérisé en ce que** la zone réactionnelle A s'étend jusqu'à une conversion de l'acroléine de 65 à 80 moles %.

3. Procédé suivant l'une des revendications 1 et 2, **caractérisé en ce que** la température de la zone réactionnelle B est d'au moins 20°C supérieure à la zone réactionnelle A.

4. Procédé suivant l'une des revendications 1 à 3, **caractérisé en ce que** la température de la zone réactionnelle A est de 245 à 260°C.

5. Procédé suivant l'une des revendications 1 à 4, **caractérisé en ce que** la température de la zone réactionnelle B est de 265 à 285°C.

6. Procédé suivant l'une des revendications 1 à 5, **caractérisé en ce que** la conversion d'acroléine est, pour un passage unique, $\geq 94$ moles %.

7. Procédé suivant l'une des revendications 1 à 6, **caractérisé en ce que** la sélectivité de la formation d'acide acrylique est $\geq 94$ moles %.

8. Procédé suivant l'une des revendications 1 à 7, **caractérisé en ce que** la charge en acroléine de la masse de catalyseur est ≥ 160 Nl/l·h.

9. Procédé suivant l'une des revendications 1 à 7, **caractérisé en ce que** la charge en acroléine de la masse de catalyseur est ≥ 170 Nl/l·h.

10. Procédé suivant l'une des revendications 1 à 9, **caractérisé en ce que** le au moins un gaz inerte est constitué pour ≥ 40 % en volume d'azote moléculaire.

11. Procédé suivant l'une des revendications 1 à 10, **caractérisé en ce que** le au moins un gaz inerte comporte de la vapeur d'eau.

12. Procédé suivant l'une des revendications 1 à 11, **caractérisé en ce que** le au moins un gaz inerte comporte du $CO_2$ et/ou du CO.

13. Procédé suivant l'une des revendications 1 à 12, **caractérisé en ce qu'**il est effectué à une pression de travail de 0,5 à 3 bars.

14. Procédé suivant l'une des revendications 1 à 13, **caractérisé en ce que** le rapport molaire $O_2$/acroléine dans le mélange de départ de gaz réactionnels est de 1 à 2.

15. Procédé suivant l'une des revendications 1 à 14, **caractérisé en ce que**, comme source d'oxygène, on utilise conjointement de l'air.

16. Procédé suivant l'une des revendications 1 à 15, **caractérisé en ce que** la teneur en acroléine du mélange de départ de gaz réactionnels est de 3 à 15% en volume.

17. Procédé suivant l'une des revendications 1 à 15, **caractérisé en ce que** la teneur en acroléine du mélange de départ de gaz réactionnels est de 5 à 8% en volume.

18. Procédé suivant l'une des revendications 1 à 17, **caractérisé en ce que** la masse active du catalyseur à lit fixe est au moins un oxyde multimétallique de la formule générale I :

$$Mo_{12}V_aX^1_bX^2_cX^3_dX^4_eX^5_fX^6_gO_n \tag{I}$$

OÙ

$X^1 =$ W, Nb, Ta, Cr et/ou Ce,
$X^2 =$ Cu, Ni, Co, Fe, Mn et/ou Zn,
$X^3 =$ Sb et/ou Bi,
$X^4 =$ un ou plusieurs métaux alcalins,
$X^5 =$ un ou plusieurs métaux alcalino-terreux,
$X^6 =$ Si, Al, Ti et/ou Zr,
a = 1 à 6,
b = 0,2 à 4,
c = 0,5 à 18,
d = 0 à 40,
e = 0 à 2,
f = 0 à 4,
g = 0 à 40, et
n = un nombre qui est déterminé par la valence et la fréquence des éléments différents de l'oxygène dans I.

19. Procédé suivant l'une des revendications 1 à 17, **caractérisé en ce que** la masse active du catalyseur à lit fixe est au moins un oxyde multimétallique de la formule générale II :

$$[D]_p[E]_q \tag{II}$$

dans laquelle les variables ont la signification suivante :

| | |
|---|---|
| D | $= Mo_{12}V_{a''}Z^1_{b''}Z^2_{c''}Z^3_{d''}Z^4_{e''}Z^5_{f''}Z^6_{g''}O_{x''}$, |
| E | $= Z^7_{12}Cu_{h''}H_{i''}O_{y''}$, |
| $Z^1$ | = W, Nb, Ta, Cr et/ou Ce, |
| $Z^2$ | = Cu, Ni, Co, Fe, Mn et/ou Zn, |
| $Z^3$ | = Sb et/ou Bi, |
| $Z^4$ | = Li, Na, K, Rb, Cs et/ou H, |
| $Z^5$ | = Mg, Co, Sr et/ou Ba, |
| $Z^6$ | = Si, Al, Ti et/ou Zr, |
| $Z^7$ | = Mo, W, V, Nb et/ou Ta, |
| a" | = 1 à 8, |
| b" | = 0,2 à 5, |
| c" | = 0 à 23, |
| d" | = 0 à 50, |
| e" | = 0 à 2, |
| f' | = 0 à 5, |
| g" | = 0 à 50, |
| h" | = 4 à 30, |
| i" | = 0 à 20, et |

x", y" = des nombres qui sont déterminés par la valence et la fréquence des éléments différents de l'oxygène dans II, et

p, q = sont des nombres différents de zéro, dont le rapport p/q est de 160/1 à 1/1,

et peut être obtenue par le fait qu'on préforme une masse d'oxyde multimétallique (E) :

$$Z^7_{\ 12}Cu_{h''}H_{i''}O_{y''} \tag{E}$$

séparément sous une forme finement divisée (masse de départ 1) et ensuite on incorpore la masse de départ 1 solide, préformée, dans une solution aqueuse, une suspension aqueuse ou un mélange sec finement divisé de sources des éléments Mo, V, $Z^1$, $Z^2$, $Z^3$, $Z^4$, $Z^5$, Z6, qui contient les éléments précités dans la stoechiométrie D :

$$Mo_{12}V_{a''}Z^1_{\ b''}Z^2_{\ c''}Z^3_{\ d''}Z^4_{\ e''}Z^5_{\ f''}Z^6_{\ g''} \tag{D}$$

(masse de départ 2) dans le rapport quantitatif souhaité p/q, on sèche le mélange aqueux éventuellement résultant, et on calcine à des températures de 250 à 600°C la masse progénitrice sèche ainsi obtenue avant ou après son séchage pour former la géométrie de catalyseur souhaitée.

20. Procédé suivant l'une des revendications 1 à18, **caractérisé en ce que** la masse de catalyseur comporte des catalyseurs annulaires.

21. Procédé suivant l'une des revendications 1 à 19, **caractérisé en ce que** la masse de catalyseur comporte des catalyseurs en forme de billes.

22. Procédé suivant l'une des revendications 1 à 21, **caractérisé en ce qu'**il est effectué dans un réacteur à faisceau tubulaire en deux zones.

23. Procédé suivant l'une des revendications 1 à 22, **caractérisé en ce que** la température de la zone réactionnelle B est d'au moins 10°C supérieure à la température de la zone réactionnelle A.

24. Procédé suivant l'une des revendications 1 à 23, **caractérisé en ce que** le mélange de départ de gaz réactionnels contient l'oxygène moléculaire et l'acroléine dans un rapport molaire de $O_2/C_3H_4O \geq 1$.

25. Procédé suivant l'une des revendications 1 à 24, **caractérisé en ce que** le catalyseur à lit fixe est une masse de catalyseur dont l'activité spécifique en volume augmente de manière continue, abrupte ou par gradins dans le sens d'écoulement par dilution par de la matière inerte du mélange de gaz réactionnels.